**Europäisches Patentamt**

**European Patent Office**

**Office européen des brevets**

(11) Veröffentlichungsnummer: **0 085 729 B1**

(12) **EUROPÄISCHE PATENTSCHRIFT**

(45) Veröffentlichungstag der Patentschrift:
**06.11.85**

(21) Anmeldenummer: **82100921.4**

(22) Anmeldetag: **09.02.82**

(51) Int. Cl.⁴: **A 61 F 13/16,** A 41 B 13/02,
A 61 F 13/00

(54) Verfahren und Vorrichtung zum Aufbringen eines hochaktiven Absorbers auf ein Substrat, insbesondere einen Hygieneartikel.

(43) Veröffentlichungstag der Anmeldung:
**17.08.83 Patentblatt 83/33**

(45) Bekanntmachung des Hinweises auf die Patenterteilung:
**06.11.85 Patentblatt 85/45**

(84) Benannte Vertragsstaaten:
**BE DE FR GB IT NL SE**

(56) Entgegenhaltungen:
**EP - A - 0 033 235**
**DE - A - 3 040 768**
**DE - B - 1 012 428**
**FR - A - 2 173 059**
**US - A - 3 399 671**
**US - A - 4 045 833**
**US - A - 4 256 526**

(73) Patentinhaber: **NORDSON CORPORATION, 555 Jackson Street P. O. Box 151, Amherst Ohio 44001 (US)**

(72) Erfinder: **Pedigrew, Colin, Immermann 20, D-4006 Erkrath 2 (DE)**

(74) Vertreter: **Eisenführ & Speiser, Martinistrasse 24, D-2800 Bremen 1 (DE)**

**Beschreibung**

Die Erfindung betrifft ein Verfahren zum Aufbringen eines hochaktiven Absorbers auf ein Substrat, insbesondere einen Hygieneartikel.

Die Erfindung betrifft weiterhin eine Vorrichtung zur Durchführung des Verfahrens zum Aufbringen eines hochaktiven Absorbers auf ein Substrat, das mehrere Bestandteile aufweist, die von Vorratspositionen in eine gemeinsame Position gebracht und dort miteinander verbunden werden.

Hygieneartikel der hier besonderes interessierenden Art sind Windeln, Damenbinden, Inkontinenzhilfen und Krankenbettunterlagen. Derartige Hygieneartikel sollen ausgeschiedene Körperflüssigkeiten über längere Zeit hinweg speichern. Die Qualität eines solchen Hygieneartikels wird deshalb wesentlich von dem Speichervermögen bestimmt. Man hat ein hohes Speichervermögen bislang im wesentlichen durch die Verwendung von saugfähigen Lagenzellstoff, Fluss-Zellulose oder Watte angestrebt. Bezogen auf ein vorgegebenes Volumen ist die Aufnahmefähigkeit dieser Materialien nicht sehr gross, so dass ein grosses Speichervermögen im wesentlichen auch nur durch ein grosses Volumen des Hygieneartikels erreicht werden konnte. Dies spielt bei Säuglingen und bettlägrigen Personen keine besondere Rolle, wird im übrigen aber als unangenehm empfunden.

Um das Speichervermögen von Hygieneartikeln zu vergrössern und/oder deren Volumen zu verringern, ist es bekannt, in die Artikel hochaktive Absorberpartikel einzubetten. Derartige Absorber können wasserunlösliche, wasserquellbare absorbierende organische Polymerisate sein, die als solche bekannt sind und die Form feinkörniger Feststoffe haben. Das Einbetten von Absorberpartikeln in die Watte o. dgl. der Artikel hat erfahrungsgemäss den Nachteil, dass sich die Partikel an einer oder mehreren Stellen des Artikels zusammenballen und ihre Speicherfunktion deshalb nur in Teilbereichen der Artikel ausüben können. In den anderen Bereichen ist die Speicherkapazität dann frühzeitig erschöpft, so dass die nachströmende Körperflüssigkeit durch den Hygieneartikel hindurchtreten kann.

Zur Vermeidung dieser Probleme ist es aus der DE-A-30 40 768 bekannt, den Absorber in einen Film einzubetten oder einen Träger mit diesem Material zu beschichten. Der Film oder Träger wird dann auf eine Windel, einen Tampon o. dgl. aufgebracht.

In dieser Weise hergestellte Hygieneartikel können das gewünschte kleine Volumen und die ebenso erwünschte hohe Speicherkapazität haben. Die Herstellung ist jedoch kostenaufwendig.

Der vorliegenden Erfindung lag deshalb die Aufgabe zugrunde, ein Verfahren und eine Vorrichtung der eingangs genannten Art vorzuschlagen, durch die sich die Herstellungskosten insbesondere von Hygieneartikeln gegenüber dem zuletzt erwähnten Stand deutlich senken lassen, ohne dass dadurch Konzessionen an die wirksame Speicherkapazität und an das Volumen gemacht werden müssten.

Das diese Aufgabe lösende Verfahren besteht darin, dass auf einen oder mehrere vorgegebene Bereiche mindestens einer Fläche des Substrates ein dünner Schmelzkleber-Film aufgebracht, auf die Fläche dann die Partikel des Absorbers gegeben und schliesslich die dabei nicht in Kontakt mit dem Schmelzkleber gekommenen überschüssigen Partikel von der Fläche wieder abgenommen werden.

Die zur Lösung der Aufgabe dienende Vorrichtung zur Durchführung des Verfahrens zum Aufbringen eines hochaktiven Absorbers auf ein Substrat, das mehrere Bestandteile aufweist, die von Vorratspositionen in eine gemeinsame Position gebracht und dort miteinander verbunden werden, besteht darin, dass vor des gemeinsamen Position oberhalb des Substrates Mittel zum Auftragen eines gleichförmig dünnen Schmelzkleber-Films auf mindestens einen vorgegebenen Bereich der Oberfläche des Substrates angeordnet, in Bewegungsrichtung des Substrates darauf folgend Mittel zum Abgeben von Absorberpartikeln an die Oberfläche des Substrates vorgesehen und hieran anschliessend vor der gemeinsamen Position Mittel zum Entfernen von überschüssigen Absorberpartikeln von der Oberfläche angeordnet sind.

Das angestrebte Ziel wird mit den genannten Mitteln aus zwei Richtungen erreicht. Zum einen lässt sich der Verbrauch an Absorberpartikeln reduzieren. Aufgrund des bekannten Aufnahmevermögens der Absorberpartikel und aufgrund statistischer Zahlen über die für einen vorgegebenen Zeitraum durchschnittlich benötigte Speicherkapazität lässt sich die benötigte Menge an Absorberpartikeln angeben. Ausserdem weiss man, dass eine Windel oder eine Binde in bestimmten Bereichen mehr Flüssigkeit aufnehmen muss als in anderen Bereichen. Somit kann man sich beim Aufbringen der Absorberpartikel auf gefährdete Zonen des Hygieneartikels beschränken. Während der Stand der Technik (DE-A-3 040 768) — soweit er zum Fixieren der Absorberpartikel Klebstoff verwendete — die gesamte Fläche der Artikel beschichtet hat und dies mit lösungsmittelhaltigen Klebstoffen tat, die auch keine einigermassen genauen Begrenzungen von absorberhaltigen Bereichen ermöglichen, lassen sich durch den Einsatz von Schmelzkleber und unter Einsatz von hierbei bekannten Applikationstechniken ausserordentlich exakt begrenzte und extrem dünne Klebstoff-Filme auftragen, die sogar eine gewisse Durchlässigkeit haben und die Flexibilität des Hygieneartikels nicht wesentlich beschränken. Folglich ist eine Einsparung an Absorbermaterial möglich, die zu einer entsprechenden Kostensenkung führt und die Qualität des Endproduktes nicht tangiert sondern eher fördert.

Das angestrebte Ziel wird zusätzlich dadurch erreicht, dass einerseits der apparative, d.h. investive Aufwand gering ist und dass vor allem die Produktionszeit gegenüber dem Stand der Technik deutlich verringert wird. Letzteres trifft in besonderem Masse dann zu, wenn ein druckempfindlicher Schmelzkleber eingesetzt wird, denn dann braucht auf die Zeit zwischen dem Auftragen des Filmes und dem Aufbringen des Absorbers keine Rücksicht genommen zu werden, so dass auch ein grösserer Spielraum für die Unterbringung der apparativen Komponenten der Vorrichtung im Rahmen existierender Geräte für die Herstellung der Hygieneartikel gegeben ist.

Weitere Merkmale und Vorteile der Erfindung ergeben sich aus der folgenden Beschreibung einiger bevorzugter Ausführungsbeispiele. In den Zeichnungen zeigen:

Fig. 1 in schematischer Darstellung eine Seitenansicht des Einlaufbereiches einer Maschine zum Herstellen von dreilagigen Binden mit einem ersten Ausführungsbeispiel einer Vorrichtung zum Aufbringen eines hochaktiven Absorbers auf eine der Lagen;

Fig. 2 eine schematische Darstellung eines abgewandelten Beispiels der Erfindung, wobei nur eine Lage eines mehrlagigen Hygieneartikels vor dessen Fertigstellung gezeigt ist;

Fig. 3 eine der Fig. 2 entsprechende Schemadarstellung eines weiter abgewandelten Ausführungsbeispiels der Erfindung;

Fig. 4 eine der Fig. 2 entsprechende abgewandelte Ausführungsform der Erfindung mit einem Tauchbad zum Ünertragen der Absorberpartikel auf eine Lage eines Hygieneartikels;

Fig. 5 eine schematische Darstellung der Steuerung der erfindungsgemässen Vorrichtung;

Fig. 6 in schematischer Darstellung eine Draufsicht auf eine Lage eines Hygieneartikels vor dessen Fertigstellung im Bereich der Vorrichtung zum Aufbringen des Absorbers;

Fig. 7 und 8 in schematischer Darstellung jeweils eine Draufsicht auf eine Lage des Hygieneartikels mit beschichteten Bereichen an unterschiedlichen Stellen der Oberfläche.

In den Zeichnungen sind die Bestandteile der Erfindung und — soweit vorhanden — die bekannten apparativen Einrichtungen zur Herstellung von Hygieneartikeln schematisch dargestellt, weil auf diese Weise das Wesen der Erfindung deutlicher hervortritt. Für einen Fachmann auf dem Gebiet der Herstellung von Hygieneartikeln oder auf dem Gebiet der Herstellung der entsprechenden Maschinen ist deren Aufbau ohnehin bekannt. Für diese Fachleute ist es lediglich notwendig zu wissen, dass und an welchen ungefähren Stellen Halterungen zur Aufnahme der Bestandteile der erfindungsgemässen Vorrichtung vorgesehen werden müssen.

Die Erfindung ist anhand von Hygieneartikeln der einleitend erwähnten Art beschrieben. Sie ist aber auch für andere Zwecke anwendbar, beispielsweise für Mullbinden zum Verbinden von Verletzungen, beispielsweise auch für Filtereinsätze oder vergleichbare Fälle im technischen Bereich. Die Verwendung des Begriffes «Hygieneartikel» soll deshalb nicht als Beschränkung verstanden werden.

Hygieneartikel bestehen im allgemeinen aus mindestens drei Lagen. Der Körperoberfläche abgewandt ist eine Schutzfolie vorgesehen, die für die Körperflüssigkeit undurchlässig ist. Hierauf folgt eine gegebenenfalls mehrteilige Lage aus Zellstoff, Flusszellulose oder Watte. Diese Kernlage übernimmt normalerweise die volle Flüssigkeitsspeicherung. Überdeckt wird die Kernlage von einem körperfreundlichen Flies oder einem Gewebe.

Im Grundsatz liegt es im Belieben des Herstellers des Hygieneartikels, auf welche Schicht oder Schichten bzw. auf welche Fläche oder Flächen der einzelnen Lagen die hochaktiven Absorberpartikel aufgebracht werden. Auszuscheiden sind lediglich die nach aussen weisenden Oberflächen der Decklage und der Wäscheschutzfolie.

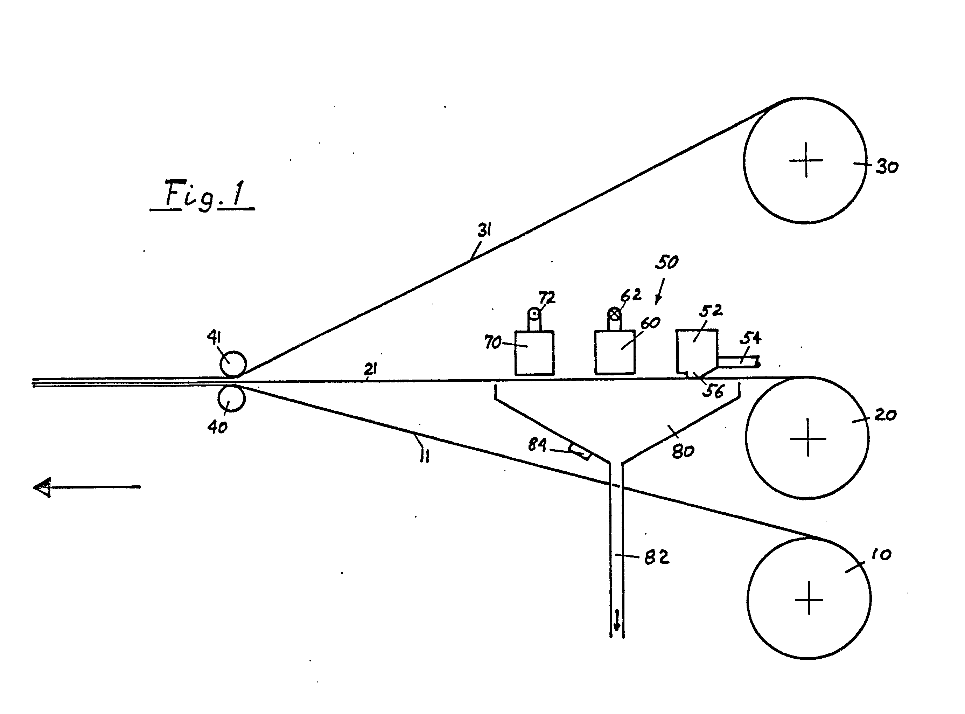

In Fig. 1 ist der Einlaufbereich einer Maschine schematisch gezeigt, die der Herstellung einer dreilagigen Binde dient. In dieser Maschine sind drei Vorratsrollen 10, 20, 30 vorgesehen, von denen die einzelnen Lagen in Richtung des Pfeiles abgezogen und gemeinsam durch zwei Druckrollen 40, 41 geführt sind. Im Bewegungsablauf nach diesen Druckrollen werden die einzelnen Lagen miteinander verbunden. Dann wird die Binde in der eingestellten Länge abgeschnitten.

Auf der Vorratsrolle 10 befindet sich die Wäscheschutzfolie 11. Auf der Vorratsrolle 20 befindet sich die Kernlage 21 der Binde und auf der Vorratsrolle 30 befindet sich ein die Decklage 31 bildendes Flies. Der Abstand zwischen den Vorratsrollen 10, 20, 30 und den Druckrollen 40, 41 kann den bei derartigen Maschinen üblichen Wert haben, weil die insgesamt mit 50 bezeichnete Vorrichtung zum Aufbringen des Absorbermaterials in Bewegungsrichtung keine grössere Länge als etwa 50 cm zu haben braucht und folglich mühelos in existierende Maschinen integriert werden kann.

Die Vorrichtung 50 weist oberhalb der Kernlage 21 und in Bewegungsrichtung gesehen zunächst mindestens einen Auftragskopf 52 für den Schmelzkleber auf. Dieser ist über eine Leitung 54 an einen nicht dargestellten Vorratstank für den Schmelzkleber und an ein Steuergerät angeschlossen, das ebenfalls nicht dargestellt ist. Der Auftragskopf 52 ist mit einer Schlitzdüse 56 versehen, die unmittelbar oberhalb der Kernlage 21 angeordnet ist.

In Bewegungsrichtung nach dem Auftragskopf 52 folgt ein Dosierkopf 60. Er hat eine in der Zeichnung nicht erkennbare, unmittelbar oberhalb der Kernlage 21 befindliche Austragöffnung für den Absorber, welcher dem Dosierkopf 60 über eine Leitung 62 von einem nicht dargestellten Vorratsbehälter zugeführt wird.

Darauf folgend ist in Bewegungsrichtung ein Saugkopf 70 oberhalb der Kernlage 21 angeordnet. Dieser hat ebenfalls eine unmittelbar oberhalb der Kernlage befindliche Saugöffnung und hat eine Breite, die grösser ist als die breiteste abzusaugende Fläche. Von dem Saugkopf 70 führt eine Saugleitung 72 zunächst zur Saugseite eines nicht dargestellten Gebläses und von dessen Druckseite weiter in den Vorratsbehälter für den Absorber.

Unterhalb der Kernlage 21 ist ein Auffangkasten 80 angeordnet. Er steht über eine Leitung 82 mit dem Vorratsbehälter für den Absorber in Verbindung, wobei in dieser Leitung 82 ebenfalls ein aus dem Auffangkasten heraussaugendes Gebläse angeordnet sein kann. Ausserdem kann an dem Auffangkasten 80 ein Vibrator 84 angeordnet sein, der die Abfuhr von Absorberpartikeln erleichtert.

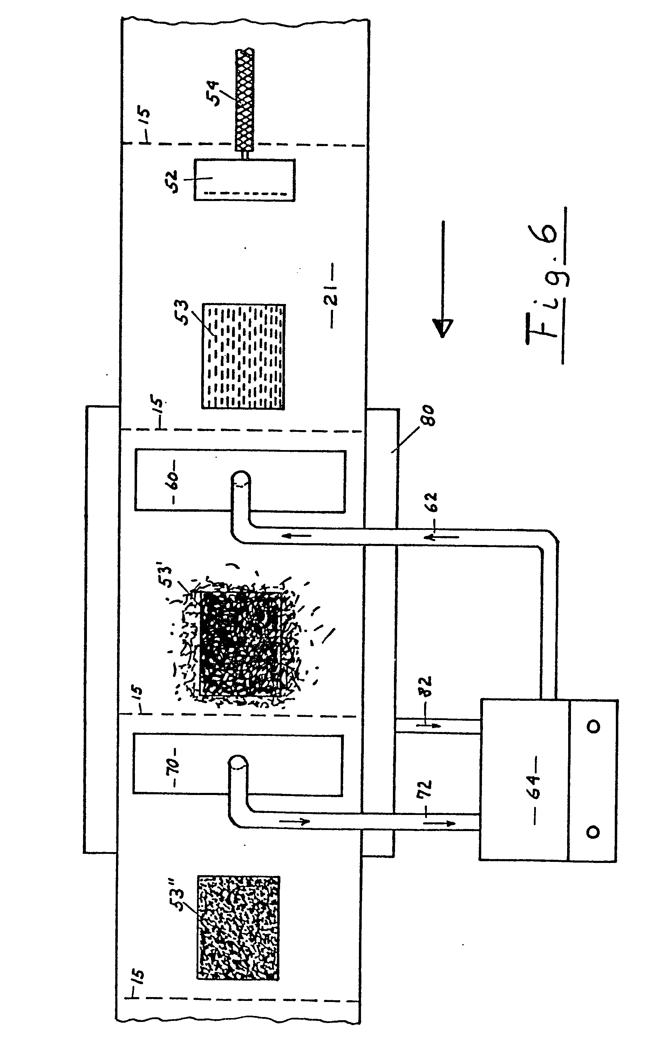

Die Arbeitsweise der Vorrichtung ist folgende: Die drei Lagen 11, 21, 31 werden kontinuierlich von den Vorratsrollen 10, 20, 30 in Richtung des Pfeiles durch die Druckrollen 40, 41 hindurch abgezogen. Je nach Länge der herzustellenden Binden oder Windeln und je nach Länge der mit dem Absorber zu versehenden Bereiche gibt das nicht gezeigte Steuergerät zunächst einen Impuls an den Auftragskopf 52, dessen Schlitzdüse sich darauf für eine vorgegebene Zeitspanne öffnet und Schmelzkleber in Form eines dünnen und scharf begrenzten Filmes auf die Kernlage 21 abgibt. Ein solcher, mit Klebstoff versehener Bereich 53 ist beispielsweise in Fig. 6 zu sehen. Vom Auftragskopf 52 wandert der Bereich 53 in Pfeilrichtung zum Dosierkopf 60, und sobald sein Anfang dort angelangt ist, wird vom Dosierkopf Absorbermaterial auf die Kernlage 21 abgegeben. Die Abgabe des Materials endet, nachdem die Hinterkante des Bereiches 53 den Dosierkopf 60 passiert hat. Die Öffnungszeiten des Dosierkopfes 60 und eine zugehörige Förderpumpe für das Absorbermaterial werden von dem Steuergerät gesteuert. Dies geschieht in bekannter Weise und braucht deshalb nicht weiter erläutert zu werden. Zu beachten ist lediglich, dass bei Verwendung eines normalen Schmelzklebers bestimmte Maximalzeiten zwischen dem Auftragen des Klebstoffes und dem Aufbringen des Absorbers nicht überschritten werden dürfen, weil der normale Schmelzkleber nur während bestimmter Zeiten eine klebrige Oberfläche aufweist. Bei Verwendung von druckempfindlichen Schmelzklebern braucht auf die Abkühlzeit keine Rücksicht genommen zu werden.

Nachdem der mit dem Klebstoff-Film versehene Bereich 53 den Dosierkopf 60 passiert hat, werden dieser Bereich sowie angrenzende Teile der Oberfläche der Kernlage 21 mit den Partikeln des Absorbers überdeckt sein, wie es in Fig. 6 mit den Bezugszeichen 53' angedeutet ist. Der Weitertransport der Kernlage 21 führt den Bereich 53' nun unter dem Saugkopf 70 hindurch. Alle überschüssigen und am Bereich 53 nicht haften bleibenden Partikel des Absorbers werden abgesaugt, so dass nach dem Passieren des Saugkopfes 70 ein scharf umrissener Bereich 53' vorliegt, der in feiner Verteilung eine vorgegebene Menge des Absorbers trägt. Die Kernlage 21 kann nun in der bisher üblichen Weise durch die Druckrollen 40, 41 hindurch zur Weiterverarbeitung in der Maschine geleitet werden.

Das vom Saugkopf 70 abgenommene Material gelangt durch die Saugleitung 72 hindurch in ein saugendes und pumpendes Fördergerät 64, in dem sich auch der nicht sichtbare Vorratsbehälter für den Absorber befindet. Angeschlossen an das Fördergerät 64 ist auch die vom Auffangkasten 80 abgehende Leitung 82.

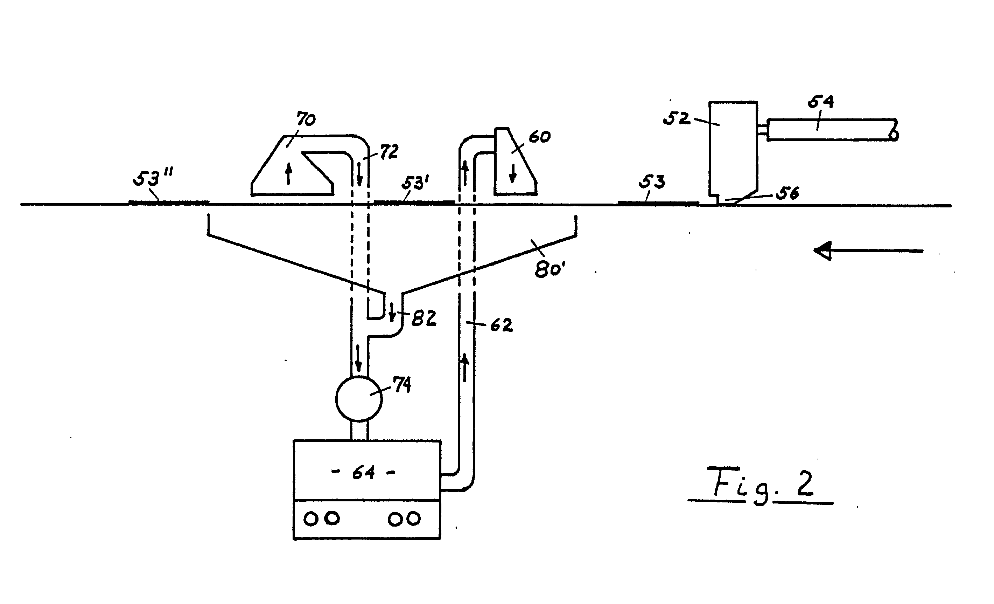

Das Ausführungsbeispiel gemäss Fig. 2 ähnelt weitgehend demjenigen der Fig. 1 Hier ist lediglich der Auffangkasten 80' in Bewegungsrichtung kürzer als zuvor dargestellt. Ausserdem zeigt die Darstellung nur den Verlauf der Kernlage 21 mit den darauf befindlichen Bereichen 53, 53', 53''. Ausserdem ist in diesem Ausführungsbeispiel ein vom Fördergerät 64 unabhängiges Gebläse 74 vorgesehen, und die beiden Leitungen 72, 82 sind vor dem Saugeingang des Gebläses 74 zusammengeführt.

Allen Ausführungsbeispielen gemeinsam ist, dass die zu beschichtende Lage (21) zumindest im Bereich des Auftragskopfes 52 nicht unterstützt wird, dort also nicht über einen Tisch oder ein Förderband läuft. Dies erleichtert das Aufbringen eines besonders gleichmässigen Filmes. Hierbei ist lediglich dafür zu sorgen, dass das Substrat im Bereich des Auftragskopfes in horizontaler Richtung möglichst präzise geführt ist.

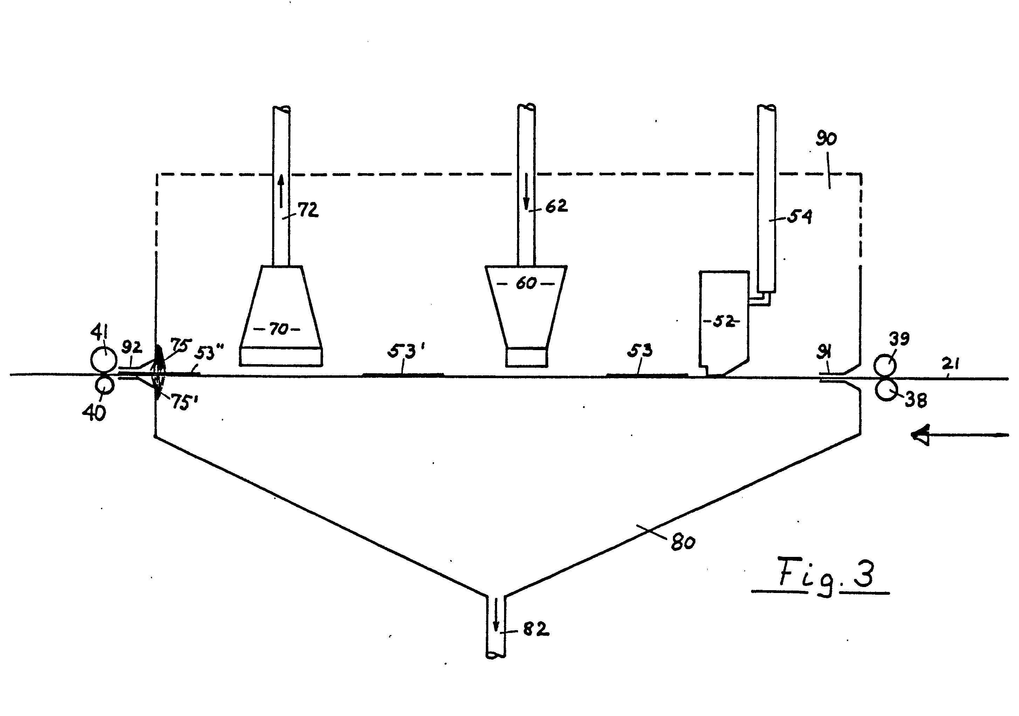

Fig. 3 zeigt eine Anordnung, bei der die erfindungsgemässe Vorrichtung 50 eine in sich abgeschlossene Baueinheit ist. Dies wird dadurch erreicht, dass dem unterhalb der Kernlage 21 befindlichen Auffangkasten 80 ein oberhalb der Kernlage 21 befindlicher Deckel 90 zugeordnet ist, so dass Auffangkasten und Deckel gemeinsam ein Gehäuse bilden. Dieses Gehäuse ist in der nicht dargestellten Maschine zwischen den Druckrollen 40, 41 und zwei davor befindlichen Druckrollen 38, 39 angeordnet. Von den Druckrollen 38, 39 gelangt die Kernlage 21 durch einen Einlassschlitz 91 hindurch in das Gehäuse hinein und verlässt dieses auf dem Weg zu den Druckrollen 40, 41 durch einen Auslassschlitz 92.

Innerhalb des Gehäuses sind in Bewegungsrichtung gesehen zunächst ein Auftragskopf 52, dann ein Dosierkopf 60 und schliesslich ein Saugkopf 70 nebst zugehörigen Leitungen 54, 62, 72 angeordnet. Die Leitungen führen in der bereits beschriebenen Weise zu einem Vorratstank für den Schmelzkleber, einem Vorratsbehälter für den Absorber und einem Steuergerät, das im übrigen mit dem Vorratstank und/oder dem Fördergerät 64 zu einer Baueinheit zusammengefasst sein kann.

Erkennbar in Fig. 3 sind die Bereiche 53, 53' und 53''. Zu beachten ist in diesem Ausführungsbeispiel im übrigen die Anwendung von Bürsten 75, 75'. Sie sind innerhalb des Gehäuses und in Bewegungsrichtung hinter dem Saugkopf 70 so angeordnet, dass sie dort die Kernlage 21 berühren und eventuell noch nicht vom Saugkopf abgesaugte Partikel des Absorbers in den Auffangkasten 80 abstreifen. Die Höhe des Decklels 90 über der Kernlage 21 wird von der Grösse der jeweils einzusetzenden Köpfe wesentlich mitbestimmt. Dies ist der Grund dafür, dass die Begrenzungslinie des Deckels 90 in Fig. 3 teilweise gestrichelt gezeichnet ist.

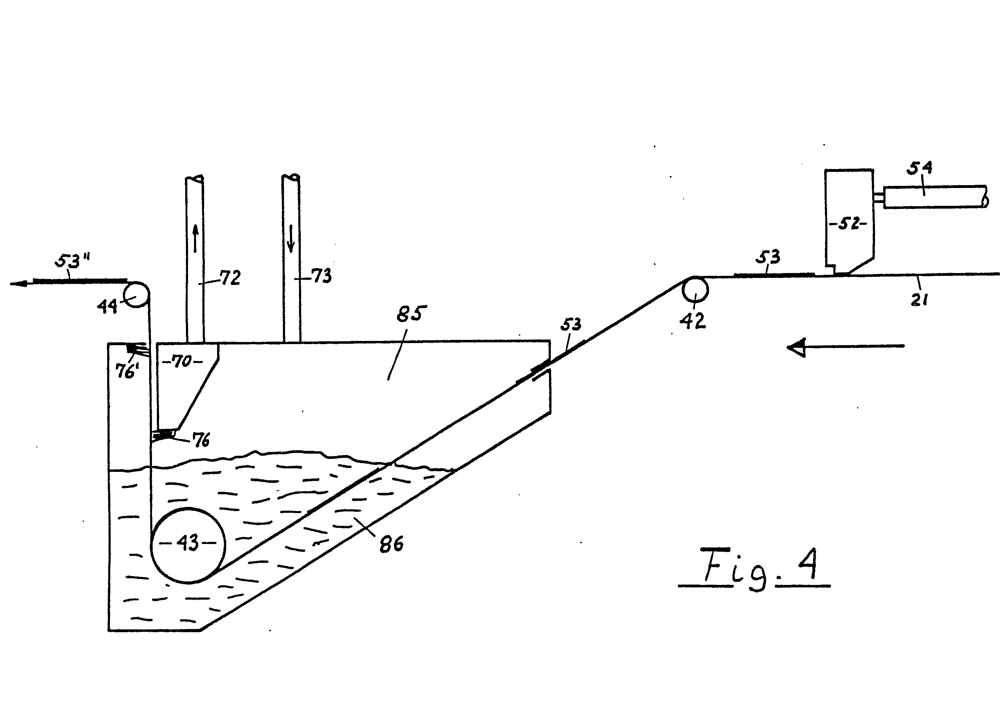

Das Ausführungsbeispiel der Erfindung gemäss Fig. 4 unterscheidet sich von den zuvor beschriebenen Beispielen dadurch, dass hier kein Dosierkopf eingesetzt wird. Die ankommende Kernlage 21 läuft zunächst unter dem Auftragskopf 52 hindurch, der auf der Oberfläche der Kernlage die bereits geschilderten Bereiche 53 erzeugt.

Nach dem Passieren des Auftragskopfes 52 wird die Kernlage 21 von einer Umlenkrolle 42 nach unten umgelenkt und gelangt in einen Behälter 85. Dieser Behälter ist bis zu einer vorgegebenen Höhe mit den Absorberpartikeln gefüllt. Eingetaucht in diese Absorberpartikel befindet sich eine weitere Umlenkrolle 43, um die die zunächst schräg nach unten bewegte Kernlage 21 nach oben umgelenkt wird. Auf diese Weise wird die in den Bereichen 53 mit dem Klebstoff-Film beschichtete Kernlage durch den Absorber hindurchgeführt. Die Bereiche 53 werden mit Absorberpartikeln bedeckt. Nach dem Verlassen des Tauchbades passiert die Kernlage 21 einen Saugkopf 70, verlässt dann den Behälter 85 und wird anschliessend mit Hilfe einer weiteren Umlenkrolle 44 in ihre ursprüngliche Richtung zurückgelenkt und gelangt schliesslich über die nicht mehr dargestellten Druckrollen zur Weiterverarbeitung in die ebenfalls nicht gezeigte Maschine.

Ein Tauchbad aus Absorberpartikeln 86 in dem Behälter 85 zum Aufbringen der Absorberpartikel auf die Bereiche 53 hat den Vorteil mechanischer Einfachheit. Allerdings werden hier beide Oberflächen der Kernlage 21 vollständig dem Absorber ausgesetzt, so dass die Kernlage nach dem Wiederauftauchen entsprechend gründlich gereinigt werden muss. Zu diesem Zweck sind Bürsten 76, 76' vorgesehen, die jeweils eine Oberfläche der Kernlage abfegen. Besonders geeignet ist der Einsatz dieses Ausführungsbeispiels dann, wenn der zu beschichtende Bestandteil des Substrates bzw. Hygieneartikels verhältnismässig glatte Oberflächen hat, durch die keine Absorberpartikel hindurchtreten können.

Die von den Bürsten 76, 76' abgekehrten Absorberpartikel fallen unmittelbar in den Absorber-Vorrat 86 im Behälter 85 zurück. Die vom Saugkopf 70 abgesaugten Partikel gelangen durch das hier nicht dargestellte Gebläse in eine Fülleitung 73 und von dort zurück in den Behälter 85. Ausserdem wird über die Fülleitung 73 frisches Absorbermaterial in den Behälter 85 nachgefüllt, wenn sich der Absorber-Spiegel innerhalb des Behälters einem vorgegebenen Minimum nähert.

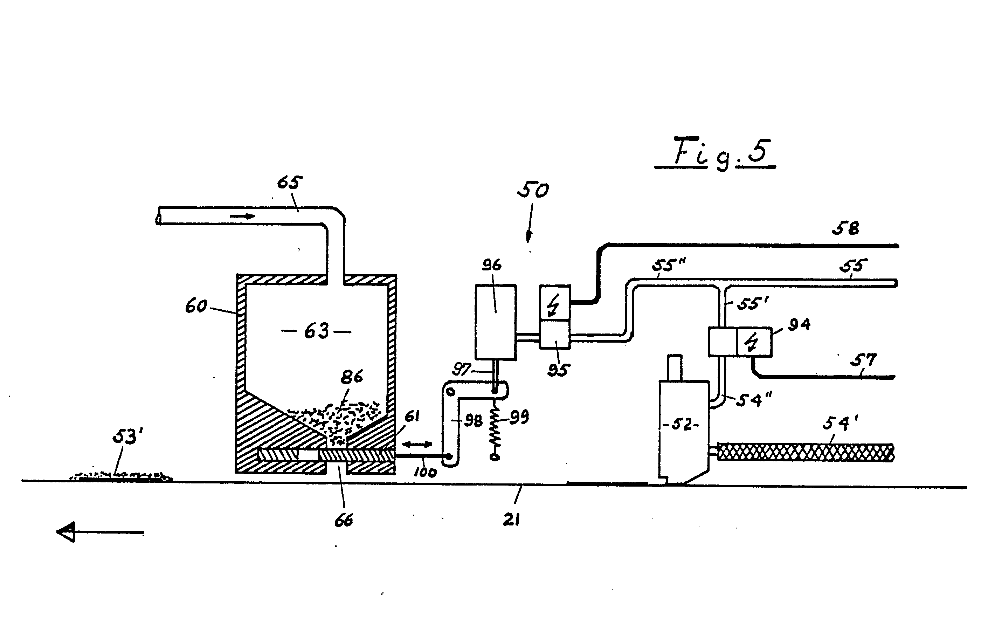

Fig. 5 zeigt ein Beispiel für die Steuerung eines Auftragskopfes 52 und eines Dosierkopfes 60. In diesem Fall ist der Auftragskopf 52 über eine Schmelzkleberleitung 54' an den nicht dargestellten Vorratstank für den Schmelzkleber angeschlossen. Von einer nicht gezeigten Druckmittelquelle kommt eine Druckmittelleitung 55 in den Bereich der Vorrichtung 50. Sie steht dauernd unter Druck. Ein zweig 55' der Druckmittelleitung ist zu einem ersten Magnetventil 94 geführt. Ein zweiter Zweig 55'' der Druckmittelleitung ist an ein zweites Magnetventil 95 angeschlossen. An den Druckmittelausgang des Magnetventils 94 ist die Leitung 54'' des Auftragskopfes 52 angeschlossen, und der Schaltmagnet des Magnetventils 94 wird von dem nicht dargestellten Steuergerät über eine Steuerleitung 57 aktiviert. In einer dem Fachmann geläufigen Weise wird der Austritt von Schmelzkleber durch die Schlitzdüse 56 des Auftragskopfes durch ein in dem Auftragskopf befindliches druckmittelbetätigtes Ventil gesteuert. In dem Augenblick, in dem der Auftragskopf beginnen soll, einen Bereich 53, d.h. einen entsprechenden Klebstoff-Film auf die Kernlage 21 abzugeben, wird von dem Steuergerät über die Leitung 57 das Magnetventil 94 geöffnet, so dass Druck von der Druckmittelleitung 55 über den Zweig 55' und die Leitung 54'' auf das innerhalb des Auftragskopfes 52 befindliche Ventil einwirken und dieses öffnen kann. Entsprechend werden das Magnetventil 94 und folglich auch das Ventil im Auftragskopf geschlossen, wenn das Steuergerät jeweils ein Ende eines Bereiches 53 über die Steuerleitung 57 signalisiert.

Der Druckmittelausgang des zweiten Magnetventils 95 ist an einen Steuerzylinder 96 angeschlossen, dessen Kolbenstange 97 einen Winkelhebel 98 verschwenkt. Der freie Arm des Winkelhebels 98 ist über eine Stange 100 an einen Schieber 61 angekoppelt, welcher im Dosierkopf 60 dessen Auslassschlitz 66 verschliesst bzw. freigibt. Dosierköpfe dieser Art sind bekannt, so dass eine detaillierte Beschreibung nicht erforderlich erscheint. Zum schnellen Verständnis sei angemerkt, dass sich in dem Dosierkopf 60 ein Vorratsraum 63 befindet, der von Zeit zu Zeit über eine Fülleitung 65 mit Absorberpartikeln 86 beschickt wird. Eine an dem Winkelhebel 98 angreifende Feder 99 spannt sowohl die Kolbenstange 97 des Steuerzylinders 96 als auch den Schieber 61 des Dosierkopfes 60 in eine Ruhestellung vor, in der der Auslassschlitz 66 des Dosierkopfes versperrt ist.

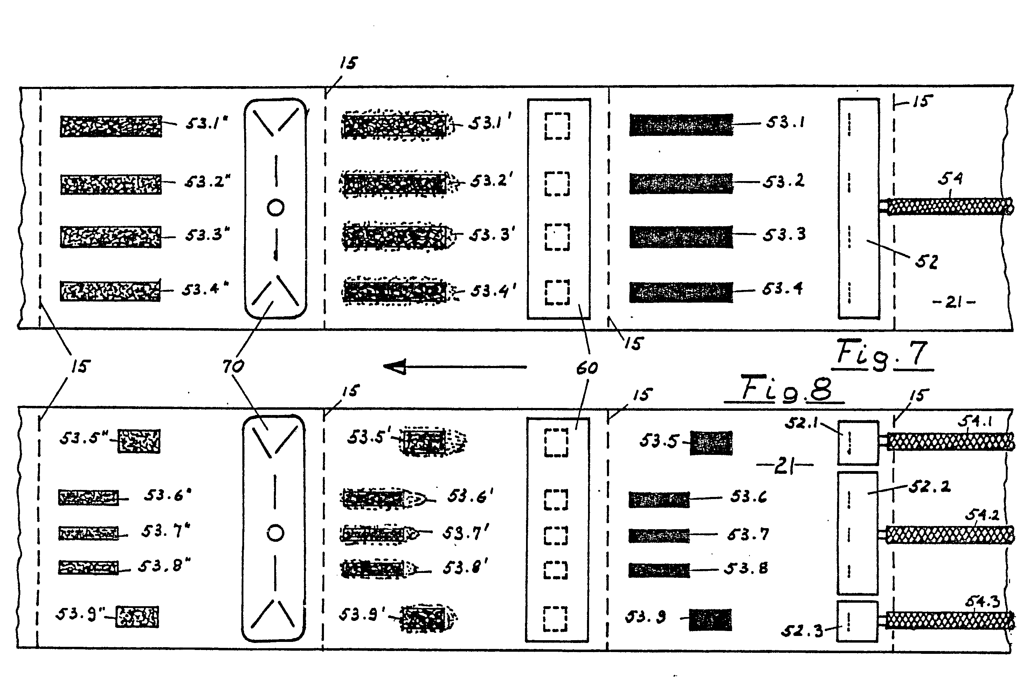

Sobald ein Klebstoff tragender Bereich 53 der Kernlage 21 in den Bereich des Schlitzes 66 des Dosierkopfes 60 kommt, gibt das nicht dargestellte Steuergerät über die Steuerleitung 58 einen Aktivierungsbefehl an das Magnetventil 95. Dieses öffnet, so dass der Druck der Druckmittelleitung 55 auf den Steuerzylinder 96 gelangt, die Kolbenstange 97 nach oben und folglich den Schieber 61 in seine geöffnete Stellung bewegt, in der Absorberpartikel 86 durch den Schlitz 66 austreten können. Der über die Steuerleitung 58 kommende Aktivierungsbefehl für das Magnetventil 95 wird vom Steuergerät beendet, sobald der gesamte Bereich 53 den Schlitz 66 passiert hat und mit Absorberpartikeln bedeckt ist. Die dadurch entstandenen Bereiche 53' wandern nun zusammen mit der Kernlage 21 weiter zu dem in Fig. 5 nicht mehr dargestellten Saugkopf 70, der keiner besonderen Steuerung bedarf, sondern im allgemeinen kontinuierlich durchläuft. Im zusammenhang mit Fig. 6 ist bereits eine der möglichen Formen der Bereiche 53 beschrieben worden. Aus der Fig. 7 und 8 geht hervor, dass gleichzeitig oder versetzt mehrere Bereiche 53.1 bis 53.4 bzw. 53.5 bis 53.9 für jeweils einen Hygieneartikel aufgetragen werden können. Die Stellen, an denen das in der Maschine fertiggestellte Erzeugnis später zur Bildung der einzelnen Hygieneartikel durchgetrennt wird, sind in den Fig. 6 bis 8 durch gestrichelte Linien 15 angedeutet.

In Fig. 7 sind von dem Auftragskopf 52 vier exakt rechteckige schmale Klebestoffbereiche 53.1 bis 53.4 hergestellt worden. Die hierzu erforderliche Konstruktion des Auftragskopfes ist dem Fachmann bekannt, so dass er keiner detaillierten Beschreibung bedarf. Erwähnt werden soll lediglich, dass in diesem Fall vier Schlitzdüsen 56 im Auftragskopf vorhanden sind und vom Steuergerät gesteuert werden.

Die Bereiche 53.5 bis 53.9 des in Fig. 8 gezeigten Musters sind in Bewegungsrichtung gegeneinander verschoben und unterscheiden sich teilweise in ihrer Grösse. Die Breite der Bereiche wird von der Breite der Schlitzdüsen 56 bestimmt, während ihre Länge vom Steuergerät über die Steuerleitung 57 (Fig. 5) unter Berücksichtigung der Vorschubgeschwindigkeit der Kernlage 21 festgelegt wird. In Fig. 8 sind drei Auftragsköpfe 52.1, 52.2, 52.3 gezeigt. Sie werden von dem Steuergerät separat gesteuert. Auf diese Weise ist sowohl das in Fig. 8 gezeigte Muster der Bereiche 53.5 bis 53.9 ebenso erreichbar wie die verschiedensten anderen Muster. Es versteht sich, dass jedem der Köpfe 52.1 bis 52.3 eine Leitung 54.1 bis 54.3 zur Versorgung mit Schmelzkleber und Steuersignalen zugeordnet ist. Ein Vergleich der Fig. 7 und 8 zeigt weiterhin, dass der Dosierkopf 60 unterschiedlich grosse Auslassöffnungen haben kann, die in diesen Figuren durch gestrichelte Linien angedeutet sind. Es ist dem Fachmann klar, dass der Austritt aus diesen Öffnungen im Falle der Fig. 7 von einem gemeinsamen Schieber gesteuert werden kann, der die mit Absorberpartikeln beschichteten Bereiche 53.1' bis 53.4 entstehen lässt. Demgegenüber benötigt man separate Schieber in dem Auftragskopf 60, wenn man die dort gezeigten Bereiche 53.5' bis 53.9' gezielt mit Absorberpartikeln beschichten will.

Alternativ kann der Absorber aber auch ungezielt auf die Kernlage 21 abgegeben werden, wie es letztlich im Ergebnis auch bei Verwendung der in Fig. 4 beschriebenen Anordnung geschehen würde.

Der die Breite der Kernlage 21 ganz übergreifende Saugkopf 70 saugt überschüssiges Absorbermaterial kontinuierlich ab, so dass letztlich die fertig beschichteten Bereiche 53.1'' bis 53.4'' (Fig. 7) bzw. 53.5'' bis 53.9'' (Fig. 8) entstehen.

Es versteht sich, dass nicht nur die durchweg erwähnte Kernlage 21 des Hygieneartikels in der erfindungsgemässen Weise mit Absorber versehen werden kann, sondern dass in gleicher Weise auch die Innenflächen aussen liegender Bestandteile des Artikels bearbeitet werden können. Darüberhinaus könnte ein mittig angeordneter Bestandteil auch beidseitig mit Absorber versehen werden. In manchen Fällen mag es dabei zweckmässig sein, die zu beschichtende Lage zu wenden, um den Klebstoff und den Absorber jeweils von oben auftragen zu können. Weiterhin liegt es in Rahmen der Erfindung, eine vergleicherweise sehr breite Lage des Artikels mit Absorberbereichen zu versehen und diese Lage dann in Längsrichtung zur Herstellung schmalerer Binden o. dgl. aufzutrennen. In solchen Fällen könnten eine entsprechende Anzahl von Auftrags- und Dosierköpfen zur Erzielung der erforderlichen Breite nebeneinander angeordnet oder ein einzelner, entsprechend breiter Auftrags- bzw. Dosierkopf benutzt werden. Im übrigen würde es auch im Rahmen der Erfindung liegen, auf das Absaugen und/oder Abbürsten der mit den Absorberpartikeln belegten Bereiche 53' ganz oder teilweise zu verzichten und die Kernlage bzw. andere beschichtete Unterlage einer Rüttelbewegung auszusetzen, die das Entfernen überschüssiger Absorberpartikel zumindest unterstützt.

**Patentansprüche**

1. Verfahren zum Aufbringen eines hochaktiven Absorbers (86) auf ein Substrat (21), insbesondere einen Hygieneartikel, dadurch gekennzeichnet, dass auf einen oder mehrere vorgegebene Bereiche (53) mindestens einer Fläche des Substrates (21) ein dünner Schmelzkleber-Film aufgebracht, auf die Fläche dann die Partikel (86) des Absorbers gegeben und schliesslich die dabei nicht in Kontakt mit dem Schmelzkleber gekommenen überschüssigen Partikel von der Fläche wieder abgenommen werden.

2. Verfahren nach Anspruch 1, dadurch gekennzeichnet, dass der Schmelzkleber ein druckempfindlicher Klebstoff ist.

3. Verfahren nach Anspruch 1 oder 2, dadurch gekennzeichnet, dass der Schmelzkleber als ein gleichförmiger, weitgehend laminarer und sehr dünner Strom in einer mit der Breite jedes Bereiches (53) übereinstimmenden Breite auf die Fläche des Substrates (21) abgelegt wird.

4. Verfahren nach einem der Ansprüche 1 bis 3, dadurch gekennzeichnet, dass das Substrat oder dessen die Fläche aufweisender Teil (21) während des Aufbringens des Schmelzklebers mindestens in den gesamten Bereichen (53) frei hängend gehalten wird.

5. Verfahren nach einem der Ansprüche 1 bis 4, dadurch gekennzeichnet, dass das Substrat oder dessen die Fläche aufweisender Teil (21) zum Aufbringen der Partikel durch einen mit Partikeln (86) gefüllten Behälter (85) hindurchgeführt wird.

6. Verfahren nach einem der Ansprüche 1 bis 4, dadurch gekennzeichnet, dass das Substrat oder dessen die Fläche aufweisender Teil (21) zum Aufbringen der Partikel (86) relativ zu einem Dosierkopf (60) bewegt wird.

7. Verfahren nach einem der Ansprüche 1 bis 6, dadurch gekennzeichnet, dass mindestens ein Teil der überschüssigen Partikel (86) durch Absaugen von der Fläche abgenommen werden.

8. Verfahren nach einem der Ansprüche 1 bis 7, dadurch gekennzeichnet, dass mindestens ein Teil der überschüssigen Partikel (86) durch Abbürsten von der Fläche abgenommen werden.

9. Verfahren nach einem der Ansprüche 1 bis 8, dadurch gekennzeichnet, dass das Substrat oder dessen die Fläche aufweisender Teil (21) zum Entfernen überschüssiger Partikel (86) einer Rüttelbewegung ausgesetzt wird.

10. Vorrichtung zur Durchführung des Verfahrens zum Aufbringen eines hochaktiven Absorbers (86) auf ein Substrat (21), insbesondere nach Anspruch 1, wobei das Substrat mehrere Bestandteile aufweist, die von Vorratspositionen (10, 20, 30) in eine gemeinsame Position (40, 41) gebracht werden, in der sie miteinander verbindbar sind, dadurch gekennzeichnet, dass vor der gemeinsamen Position oberhalb des Substrates (21) Mittel (52) zum Auftragen eines gleichförmig dünnen Schmelzkleber-Films auf mindestens einen vorgegebenen Bereich (53) der Oberfläche des Substrates angeordnet, in Bewegungsrichtung des Substrates darauf folgend Mittel (60, 85) zum Abgeben von Absorberpartikeln (86) an die Oberfläche des Substrates vorgesehen und hieran anschliessend vor der gemeinsamen Position Mittel (70, 75, 76) zum Entfernen von überschüssigen Partikeln von der Oberfläche des Substrates angeordnet sind.

11. Vorrichtung nach Anspruch 10, dadurch gekennzeichnet, dass die Mittel zum Auftragen eines gleichförmig dünnen Schmelzkleber-Films aus einem Auftragskopf (52) mit einer gesteuerten Schlitzdüse (56) bestehen, der unmittelbar oberhalb des zu beschichtenden Substrates angeordnet und über Leitungen (54, 54', 54'') an einen Vorratstank für den Schmelzkleber und an ein Steuergerät zur Steuerung der Schmelzkleberabgabe durch die Schlitzdüse angeschlossen ist.

12. Vorrichtung nach Anspruch 11, dadurch gekennzeichnet, dass in Richtung der Breite des Substrates (21) mehrere Auftragsköpfe (52) nebeneinander angeordnet sind.

13. Vorrichtung nach einem der Ansprüche 10, 11 oder 12, dadurch gekennzeichnet, dass die Mittel zum Abgeben von Absorberpartikeln (86) an die Oberfläche aus einem Dosierkopf (60) bestehen, der mindestens eine der zu beschichtenden Fläche des Substrates (21) zugeordnete steuerbare Auslassöffnung (66) aufweist und zum Takten der Auslassöffnung(en) an das Steuergerät angeschlossen ist.

14. Vorrichtung nach Anspruch 13, dadurch gekennzeichnet, dass der Dosierkopf (60) in Richtung der Breite des Substrates (21) mehrere separate Auslassöffnungen (66) aufweist, die gemeinsam oder unabhängig voneinander aufsteuerbar sind.

15. Vorrichtung nach Anspruch 13 oder 14, dadurch gekennzeichnet, dass die Auslassöffnungen (66) des Dosierkopfes (60) pneumatisch steuerbar sind.

16. Vorrichtung nach einem der Ansprüche 10 bis 12, dadurch gekennzeichnet, dass die Mittel zum Abgeben von Absorberpartikeln (86) an die Oberfläche aus einem Behälter (85) bestehen, der teilweise mit Absorberpartikeln gefüllt ist, einen Einlass für das Substrat (21) sowie einen Auslass und unterhalb der Füllstandshöhe der Absorberpartikel eine Umlenkeinrichtung (43) aufweist, derart, dass das Substrat innerhalb des Behälters in die Absorberpartikel hinein und aus diesen wieder heraus bewegbar ist und dass in Bewegungsrichtung des Substrates vor dem Auslass des Behälters die Mittel (70, 76) zum Entfernen von überschüssigen Absorberpartikeln angeordnet sind.

17. Vorrichtung nach einem der Ansprüche 10 bis 16, dadurch gekennzeichnet, dass die Mittel zum Entfernen von überschüssigen Absorberpartikeln (86) von der Oberfläche mindestens einen Saugkopf (70) aufweisen, der eine den mit Absorberpartikeln belegten Bereichen (53') zugekehrte Öffnung aufweist und an die Saugseite eines Gebläses (74) angeschlossen ist und dass die Druckseite des Gebläses mit einem Vorratsbehälter für die Absorberpartikel verbunden ist.

18. Vorrichtung nach Anspruch 17 oder nach den Ansprüchen 10 bis 16, dadurch gekennzeichnet, dass die Mittel zum Entfernen von überschüssigen Absorberpartikeln (86) von der Oberfläche aus Bürsten (75, 76) bestehen oder solche aufweisen und dass diese Bürsten an jeder Oberfläche des Substrates (21) anliegen, die von überschüssigen Absorberpartikeln zu befreien ist.

19. Vorrichtung nach einem der Ansprüche 10 bis 15, 17 oder 18, dadurch gekennzeichnet, dass unterhalb der Fläche des Substrates (21), die den Mitteln (60) zum Abgeben von Absorberpartikeln (86) zugekehrt ist, sich ein Auffangkasten (80) zum Auffangen von überschüssigen Absorberpartikeln befindet.

20. Vorrichtung nach einem der Ansprüche 10 bis 19, dadurch gekennzeichnet, dass die Mittel zum Auftragen des Schmelzkleber-Films, zum Abgeben von Absorberpartikeln und zum Entfernen überschüssiger Absorberpartikel in einem gemeinsamen Gehäuse (80, 90) angeordnet sind und dieses mit Anschlüssen für die Ver- und Entsorgung der Mittel sowie deren Steuerung versehen ist.

## Claims

1. Process for the application of a highly absorbent material (86) onto a substrate (21), in particular a hygiene article, characterized in that a thin film of fusion adhesive is applied onto one or more predetermined zones (53) of at least one surface of the substrate (21), the particles (86) of the absorbent material being then added onto the surface and finally the surplus particles which have not thereby come into contact with the fusion adhesive being removed from the surface.

2. Process according to Claim 1, characterized in that the fusion adhesive is a pressure-sensitive adhesive material.

3. Process according to Claim 1 or 2, characterized in that the fusion adhesive is deposited onto the surface of the substrate (21) as a uniform, largely laminar and very thin stream at a width which coincides with the width of each zone (53).

4. Process according to one of Claims 1 to 3, characterized in that the substrate or its part (21) which has the surface, is held freely suspended during the application of the fusion adhesive, at least in all the zones (53).

5. Process according to one of Claims 1 to 4, characterized in that the substrate or its part (21) having the surface, is passed through a container (85) filled with particles (86) for the application of the particles.

6. Process according to one of Claims 1 to 4, characterized in that the substrate or its part (21) having the surface, is moved relatively to a dosaging head (60) for the application of the particles (86).

7. Process according to one of Claims 1 to 6, characterized in that at least a part of the surplus particles (86) is removed from the surface by suction.

8. Process according to one of Claims 1 to 7, characterized in that at least a part of the surplus particles (86) is removed by being brushed off the surface.

9. Process according to one of Claims 1 to 8, characterized in that the substrate or its part (21) having the surface, is subjected to a shaking motion for removal of the surplus particles (86).

10. Apparatus to carry out the process for the application of a highly absorbent material (86) onto a substrate (21), particularly according to Claim 1, in which the substrate has several component parts, which are brought from supply positions (10, 20, 30) into a common position (40, 41), in which they are able to be combined together, characterized in that in front of the common position above the substrate (21), means (52) are arranged for the application of a uniformly thin film of fusion adhesive onto at least one predermined zone (53) of the surface of the substrate, in the direction of movement of the substrate following thereafter means (60, 85) are provided for the depositing of particles (86) of absorbent material onto the surface of the substrate and following on from this, in front of the common position, means (70, 75, 76) are arranged for the removal of surplus particles from the surface of the substrate.

11. Apparatus according to Claim 10, characterized in that the means for the application of a uniformly thin film of fusion adhesive consist of an applicator head (52) with a controlled slotted nozzle (56), which is arranged immediately above the substrate which is to be coated and which is connected via ducts (54, 54', 54'') to a storage tank for the fusion adhesive and to a control device to control the delivery of fusion adhesive through the slotted nozzle.

12. Apparatus according to Claim 11, character-ized in that in the direction of the width of the substrate (21) several applicator heads (52) are arranged adjacent to each other.

13. Apparatus according to one of Claims 10, 11 or 12, characterized in that the means for the depositing of particles (86) of absorbent material onto the surface consist of a dosaging head (60), which has at least one controllable outlet opening (66) associated with the surface of the substrate (21) which is to be coated, and which is connected to the control device for the rhythmic timing of the outlet opening(s).

14. Apparatus according to Claim 13, characterized in that the dosaging head (60) has several separate outlet openings (66) in the direction of the width of the substrate (21), which are able to be controlled together or independently of each other.

15. Apparatus according to Claim 13 or 14, characterized in that the outlet openings (66) of the dosaging head (60) are able to be controlled pneumatically.

16. Apparatus according to one of Claims 10 to 12, characterized in that the means for the depositing of particles (86) of absorbent material onto the surface consist of a container (85), which is partially filled with particles of absorbent material, has an inlet for the substrate (21) and an outlet and beneath the full level of the particles of absorbent material has a deflection mechanism (43), such that the substrate within the container is able to be moved into the particles of absorbent material and out from them again and that in the direction of movement of the substrate in front of the outlet of the container, the means (70, 76) for the removal of surplus particles of absorbent material are arranged.

17. Apparatus according to one of Claims 10 to 16, characterized in that the means for the removal of surplus particles (86) of absorbent material from the surface have at least one suction head (70), which has an opening which faces the zones (53') covered with particles of absorbent material and which is connected to the suction side of a blower (74) and that the pressure side of the blower is connected with a storage container for the particles of absorbent material.

18. Apparatus according to Claim 17 or according to Claims 10 to 16, characterized in that the means for the removal of surplus particles (86) of absorbent material from the surface consist of brushes (75, 76) or have the latter, and that these brushes rest against each surface of the substrate (21), which is to be freed of surplus paricles of absorbent material.

19. Apparatus according to one of Claims 10 to 15, 17 or 18, characterized in that beneath the surface of the substrate (21), which faces the means (60) for the delivery of particles (86) of absorbent material, there is a collecting box (80) to collect surplus particles of absorbent material.

20. Apparatus according to one of Claims 10 to 19, characterized in that the means for the application of the film of fusion adhesive, for the delivery of particles of absorbent material and for the removal of surplus particles of absorbent material, are arranged in a common housing (80, 90), and the latter is provided with connections for supply to and removal from the means and the control thereof.

## Revendications

1. Procédé pour l'application d'un matériau très fortement absorbant (86) sur un substrat (21), notamment un article hygiénique, caractérisé en ce que, sur une ou plusieurs régions prédéterminées (53) d'au moins une surface de substrat (21), on dépose une mince pellicule d'une colle à fusion, on dépose ensuite sur la surface les particules (86) de matériau absorbant et on élimine enfin de la surface les particules excédentaires qui ne sont pas venues en contact avec la colle à fusion.

2. Procédé selon la revendication 1, caractérisé en ce que la colle à fusion est une colle sensible à la pression.

3. Procédé selon la revendication 1 ou la revendication 2, caractérisé en ce que la colle à fusion est déposée sur la surface du substrat (21) sous forme d'un film uniforme, dans une large mesure laminaire et très mince suivant une largeur coïncidant avec la largeur de chaque région (53).

4. Procédé selon l'une des revendications 1 à 3, caractérisé en ce que le substrat ou sa partie (21) peésentant la surface est maintenu librement suspendu pendant l'application de la colle en fusion au moins dans la totalité des régions (53).

5. Procédé selon l'une des revendications 1 à 4, caractérisé en ce que le substrat ou sa partie (21) présentant la surface est acheminé pour l'application des particules au travers d'un récipient (85) rempli de particules (86).

6. Procédé selon l'une des revendications 1 à 4, caractérisé en ce que le substrat ou sa partie (21) présentant la surface est pour l'application des particules (86) déplacé par rapport à une tête de dosage (60).

7. Procédé selon l'une des revendications 1 à 6, caractérisé en ce qu'au moins une partie des particules excédentaires (86) est éliminée de la surface par aspiration.

8. Procédé selon l'une des revendications 1 à 7, caractérisé en ce qu'au moins une partie des particules excédentaires (86) est éliminée de la surface par brossage.

9. Procédé selon l'une des revendications 1 à 8, caractérisé en ce que le substrat ou sa partie (21) présentant la surface est soumis pour l'élimination des particules excédentaires (86) à un movement à secousses.

10. Dispositif pour la mise en oeuvre du procédé pour l'application d'un matériau très fortement absorbant (86) sur un substrat (21), notamment selon la revendication 1, le substrat comportant plusieurs éléments constituants amenés à partir de postes de stockage (10, 20, 30) à un poste commun (40, 41) où ils sont liés entre eux, caractérisé en ce que des moyens (52) pour l'application d'une mince pellicule uniforme d'une colle à fusion sut au moins une région prédéterminée (53) de la surface du substrat sont disposés avant le poste commun au-dessus du substrat (21), des moyens (60, 85) pour le dépôt des particules de matériau absorbant (86) sur la surface du substrat étant prévus à la suite dans le sens de déplacement du substrat et des moyens (70, 75, 76) pour l'élimination des particules de matériau absorbant excédentaires étant prévus immédiatement ensuite avant le poste commun.

11. Dispositif selon la revendication 10, caractérisé en ce que les moyens pour l'application d'une mince pellicule uniforme de colle à fusion sont constitués d'une tête d'enduction (52) avec une buse à fente commandé (56), tête qui est disposée immédiatement au-dessus du substrat à revêtir et qui est raccordée par des conduites (54, 54', 54'') à un réservoir de stockage de colle à fusion ainsi qu'à un appareil de commande de la fourniture de colle à fusion à travers la fente à buse.

12. Dispositif selon la revendication 11, caractérisé en ce que plusieurs têtes d'enduction (52) sont disposées l'une à côté de l'autre dans le sens de la largeur du substrat (21).

13. Dispositif selon l'une des revendications 10, 11 ou 12, caractérisé en ce que les moyens pour le dépôt des particules de matériau absorbant (86) sur la surface sont constitués par une tête doseuse (60), présentant au moins une ouverture de sortie (66) commandée et associée à la surface du substrat à revêtir (21), cette ouverture étant raccordée à l'appareil de commande pour la commande cyclique de l'ouverture ou des ouvertures de sortie (66).

14. Dispositifs selon la revendication 13, caractérisé en ce que la tête doseuse (60) présente dans le sens de la largeur du substrat (21), plusieurs ouvertures de sortie séparées (66), dont l'ouverture peut être commandée conjointement ou indépendamment l'une de l'autre.

15. Dispositif selon la revendication 13 ou la revendication 14, caractérisé en ce que les ouvertures de sortie (66) de la tête doseuse (60) sont commandées par voie pneumatique.

16. Dispositifs selon l'une des revendications 10 à 12, caractérisé en ce que les moyens pour le dépôt des particules de matériau absorbant (86) sur la surface sont constitués par un récipient (85), partiellement rempli de particules de matériau absorbant, comportant une entrée pour le substrat (21) ainsi qu'une sortie et, au dessous du niveau de remplissage de particules de matériau absorbant, un organe de renvoi (43), de sorte que le substrat peut être déplacé à l'intérieur du récipient dans les particules de matériau absorbant et peut sortir hors de celles-ci et que, dans le sens de déplacement du substrat, les moyens (70, 76) pour l'élimination des particules de matériau absorbant excédentaires sont disposés avant la sortie du récipient.

17. Dispositif selon l'une des revendications 10 à 16, caractérisé en ce que les moyens pour l'élimination des particules de matériau absorbant excédentaires (86) de la surface comportent au moins une tête d'aspiration (70) qui présente une ouverture dirigée vers les régions (53') recouvertes de particules de matériau absorbant et qui est raccordée au côté d'aspiration d'un ventilateur et en ce que le côté de refoulement du ventilateur est relié à un réservoir de stockage de particules de matériau absorbant.

18. Dispositif selon la revendication 17 ou selon l'une des revendications 10 à 16, caractérisé en ce que les moyens pour l'élimination des particules de matériau absorbant excédentaires (86) de la surface sont constituée par des brosses (75, 76) ou comportent de telles brosses et en ce que ces brosses s'appliquent sur chacune des surfaces du substrat (21) que l'on doit débarrasser des particules de matériau absorbant excédentaires.

19. Dispositif selon l'une des revendications 10 à 15, 17 ou 18, caractérisé en ce qu'un caisson collecteur (80) pour recueillir les particules de matériau absorbant excédentaires se trouve au-dessous de la surface du substrat (21) dirigée vers les moyens (60) pour le dépôt des particules de matériau absorbant (86).

20. Dispositif selon l'une des revendications 10 à 19, caractérisé en ce que les moyens pour l'application de la pellicule de colle à fusion, pour le dépôt des particules de matériau absorbant et pour l'élimination des particules de matériau absorbant excédentaires sont disposés dans un carter commun (80, 90) et celui-ci est muni de raccords pour l'alimentation et l'évacuation des produits ainsi que pour la commande.

Fig.1

Fig. 2

Fig. 3

Fig. 4

0 085 729

Fig. 5

Fig. 6

Fig. 7

Fig. 8